# EUROPEAN PATENT APPLICATION

(11) **EP 4 751 683 A1**
(43) Date of publication of application: **03.06.2026**
(21) Application number: 24216974.6
(22) Date of filing: 02.12.2024
(51) Int. Cl.: A61F 2/24

(54) **ADHESIVE STENT-VALVE CONNECTION**

(71) Applicant: GrOwnValve GmbH, 13353 Berlin (DE)
(72) Inventor: KORIATH, Max, 13817 Berlin (DE); BAUMBACH, Hardy, 70376 Stuttgart (DE); NISSL, Thomas, 37318 Mackenrode (DE); KRENKLER, Elisabeth, 10245 Berlin (DE); WARNACK, Boris, 4125 Riehen (CH); KOSCHE, Florence, 13088 Berlin (DE); WENTZEL, Carlo, 10409 Berlin (DE); WEBER, Eugenia, 10711 Berlin (DE); SCHMITT, Boris, 10435 Berlin (DE); MODOLELL, Jordi, 17192 Berlin (DE)
(74) Representative: Hepp Wenger Ryffel AG

(57) **Abstract**

The invention relates to a medical implant (1) such as an implantable valve. The medical implant (1) comprises a main body (2) and a leaflet assembly (5). The leaflet assembly (5) is formed separate from the main body (2) and is attachable or attached thereto.

## Description

The present invention relates to medical implants and related methods according to the preamble of the independent claims.

It known in the prior art to replace body parts with implants. For example, defective heart valves may be replaced with heart valve prostheses.

An example of a heart valve prosthesis is disclosed in US 2004/024451 A1.

More recently, heart valve prosthesis made individually for a certain patient and based on the patient's own tissue were disclosed.

For example, US 2024/041593 A1 discloses a cardiac valve prosthesis which is obtainable by providing human or animal body tissue and shaping the body tissue in a shaping process, and fixation and stabilization of the body tissue by a cross-linking agent.

US 2024/024098 A1 method for manufacturing a mold for a cardiac valve prosthesis.

US 2024/041593 A1 and US 2024/024098 A1 are incorporated herein by reference.

The heart valves and methods known in the prior art present certain disadvantages. For example, leaflets which are present in heart valve prostheses typically need to be mounted on a stent structure, which is often time-consuming, error-prone, and time-consuming. Thus, when a valve is made from a patient's own tissue, the patient may have to undergo several procedures in between which the valve is manufactured, or remain in surgery for extended times until the valve is manufactured from collected tissue.

Thus, the object of the present invention is to overcome the drawbacks of the prior art, in particular to provide an implant which can be assembled more easily and quickly, for example so as to improve the treatment of patients when such implants are made from a patient's own tissue.

This and other objects are achieved by the medical implant and the methods according to the characterizing portion of the independent claims of the invention.

The medical implant according to the invention is preferably an implantable valve, for example a prosthetic heart valve. The medical implant comprises a main body and a leaflet assembly. The leaflet assembly is formed separately from the main body, i.e., not integrally, and is attachable or attached to the main body.

The main body may be a stent.

The main body may comprise or consist of a material selected from: a shape memory alloy, a resorbable metal alloy, a bimetal, a magnesium alloy, an iron alloy, a cobalt alloy, a chromium alloy, a polymer (such as, for example, polylactide). Any combination of one or more of these materials is also conceivable.

The main body may be adapted to be placed in one of an aortic valve, a mitral valve, a pulmonary valve, and/or a tricuspid valve.

In particular, the main body may have a size, a diameter across a flow opening, a length along a flow opening axis, and/or a shape corresponding to any of the above valves in a human or animal patient.

The leaflet assembly may be an autologous leaflet assembly and at least partially formed from human and/or animal tissue (e.g. from a patient's own tissue). To this end, the leaflet assembly may be obtainable by a process comprising the steps of providing human or animal body tissue; shaping the body tissue in a shaping process to give the body tissue a shape and size of a cardiac valve; fixation and stabilization of the body tissue by a cross-linking agent, thereby preserving the shape given to the body tissue by the shaping process.

The term "body tissue" in this context may, in particular, include connective tissue, muscular tissue, nervous tissue, epithelial tissue, fascial tissue, peritoneal tissue, and cardiac tissue.

The method by which the leaflet assembly may be obtainable makes use of a shaping process in which a desired shape and size, namely the shape and size of a cardiac valve, may be given to the body tissue. The cross-linking agent may then preserve this given shape.

The shaping process may be a process in which positive or negative pressure is applied to the body tissue. For example, the body tissue may be inserted into a mold and deformed by applying pressure, e.g., by a suction process. The body tissue may then adapts the shape of the mold. A suitable mold as disclosed in US 2024/024098 A1.

In an embodiment, the shaping process may be a deep drawing process, e.g., a deep drawing process using an individually shaped forming die.

Cross-linking may not only preserve the given shape of the body tissue, but may also serve for stabilizing the structure of the extracellular matrix of the body tissue, wherein the extracellular matrix may comprises glycosaminoglycans, collagen and elastin.

The cross-linking agent comprises or consists of at least one secoiridoid as disclosed in US 2024/041593 A1.

Alternatively, the leaflet assembly may also be a leaflet assembly manufactured from other synthetic or natural materials, such as bovine pericardium. Still, the medical implant according to the invention provides advantages in that a leaflet assembly may be selected (e.g. one with a suitable size, or from a suitable material, or one from several having different features) for a specific patient, e.g. during surgery, and rapidly and reliably assembled with a main body such as a stent.

Preferably, the main body is coated with a material selected from electrospun fibers, polymers, synthetic tissues, pericardium, peritoneum, fascia lata, and/or a material applied by an electrospinning and/or dip coating and/or spray coating process, and/or a combination thereof. Thereby, a main body coating may be arranged on the main body.

The coating may provide an intermediate layer to facilitate attachment of the leaflet assembly. For example, the coating may provide a primer for an adhesive connection.

Alternatively, the main body may be connected with its bare surface to the leaflet assembly.

However, it is also conceivable to only coat part of the main body and leave another part bare.

Preferably, the leaflet assembly is attachable or attached to the main body and/or the main body coating by an adhesive connection.

An adhesive connection is particularly advantageous because it is easy to provide. The adhesive connection is secure and can be applied in a fast way. Therefore, a surgeon may assemble the leaflet assembly to the main body in a short time during the surgery, for example directly after obtaining a patient's tissue and forming a leaflet assembly therefrom. A second surgery may be avoided, and the waiting time for the patient in the surgery may be reduced.

The adhesive connection may be formed by an adhesive selected from methacrylated gelatine (GelMA), a cyanoacrylate, a fibrin-based adhesive, an adhesive powder, a collagen-based adhesive, and/or any combination thereof.

These adhesives provide a secure connection without extended drying or curing times. In addition, these adhesives are biologically compatible and are available in medical grades.

The invention is further directed to a method of producing a medical implant. The medical implant may be a medical implant as described herein above. The method comprises the steps of providing a main body, providing a leaflet assembly, and attaching the leaflet assembly to the main body. Preferably, the leaflet assembly is attached to the main body by glueing, e.g. e.g. using an adhesive composition as described herein above. The leaflet assembly is preferably an autologous leaflet assembly.

Preferably, the method further comprises a step of producing a leaflet assembly by providing human or animal body tissue; shaping the body tissue in a shaping process to give the body tissue a shape and size of a cardiac valve; fixation and stabilization of the body tissue by a cross-linking agent, thereby preserving the shape given to the body tissue by the shaping process.

The adhesive composition may be delivered via perforated balloon and/or a perforated lumen.

A balloon may be used to deliver the adhesive composition to an inside of the main body evenly.

Additionally or alternatively, the adhesive composition may be provided in a syringe with which the adhesive composition is injected into said lumen and/or balloon.

To this end, a balloon may be provided which comprises a Luer lock interface for connection to a syringe.

The method may further comprise a step of curing the adhesive composition. To this end, the adhesive composition may be curable and may, optionally, be two-component adhesive.

Curing may include irradiation with light (e.g. UV light, blue light, etc.). Other curing mechanisms are conceivable as well, for example an increase in temperature (which may be achieved by providing a heated liquid through a balloon, through IR irradiation, etc.), through contact with a further substance (e.g. a crosslinker, water, a solvent, etc.). Of course, two or more curing mechanism may also be combined.

The invention is further directed to a method of treating a patient. The method comprises the steps of obtaining body tissue from a patient to be treated, shaping the body tissue in a shaping process to give the body tissue a shape and size of a leaflet assembly, fixating and stabilizing the body tissue by a cross-linking agent and thereby preserving the shape given to the body tissue by the shaping process. Furthermore, the leaflet assembly is attached to an implant main body. Preferably, the attachment is made by glueing. Thereby, a medical implant is formed, which may be a medical implant as described herein above. The implant is implanted in the patient.

The medical implant is preferably a prosthetic cardiac valve. The medical implant may be implanted in the patient at a native cardiac valve.

Preferably, the implant main body is removed after implantation.

For example, the implant main body may be resorbable and dissolve at the implant site after a certain time frame. Additionally or alternatively, the implant main body may be caused to disintegrate, e.g. by sound waves.

In the following, the invention is described in detail with reference to the following figures, showing:
- Fig. 1:: schematically medical implant in a cross-sectional view.
- Figs. 2a-2c:: a mold which may be used to produce a leaflet assembly.
- Fig. 3a-3b:: schematically a way of applying a pressure to an implant.
- Fig. 4:: schematically a mold which may be used to apply an adhesive composition.
- Fig. 5:: schematically a second way of applying an adhesive composition.
- Fig. 6:: schematically a third way of applying an adhesive composition.

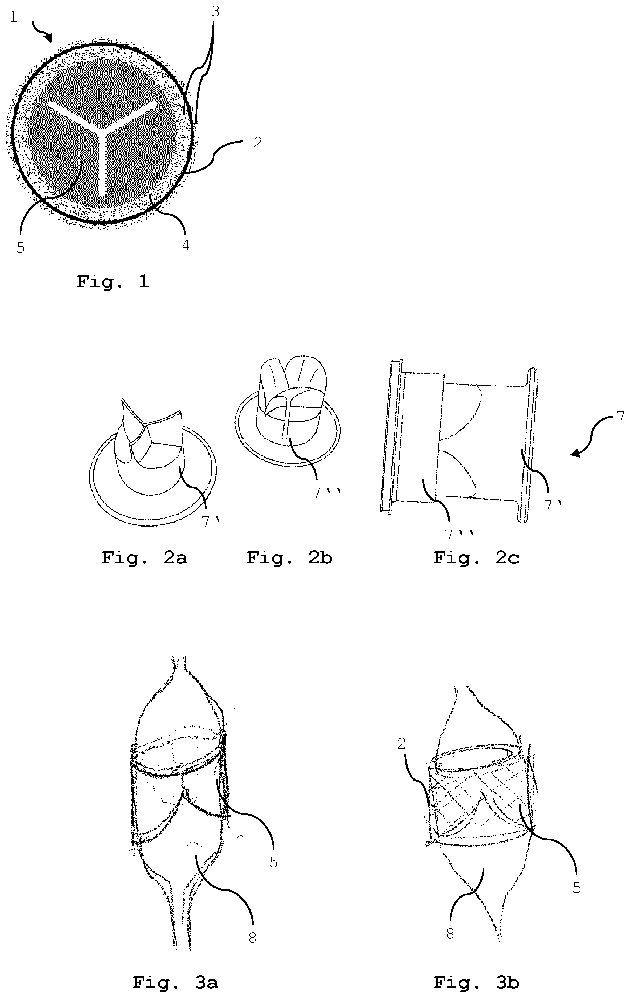

Fig. 1 shows schematically a medical implant 1, here in the form of a prosthetic valve (for example, an aortic valve) in a cross-sectional top view. The medical implant 1 comprises a leaflet assembly 5 which is assembled in main body 2 formed by a stent structure. The main body 2 is coated with electrospun fibers 3 both on inside and outside surfaces, providing better adhesion of the adhesive composition 4 which attaches the leaflet assembly 5 to the main body.

Fig. 2a shows a first part 7' of a mold.

Fig. 2b shows a second part 7" of a mold.

Fig. 2c shows the first part 7' and the second part 7" of Figs. 2a-2b in an assembled state forming a mold 7. A patient's tissue, e.g. pericardium, may be placed in the mold and formed into a leaflet assembly. Subsequently, the leaflet assembly may be attached to a main body. As will be described herein below, in some embodiments, the attachment of the leaflet assembly to the main body may be done while the leaflet assembly is still in the mold. In some cases, the adhesive assembly may be delivered via the mold. In other embodiments, the leaflet assembly is removed from the mold and attached to the main body subsequently, for example using an adhesive.

As shown in Fig. 3a, a balloon 8 may be arranged in an inner channel of the leaflet assembly 5. Thereby, a pressure may be exerted on the leaflet assembly 5 in an outside direction, through which the leaflet assembly 5 is pushed against the stent structure 2, as shown in Fig. 3b.

In general, it will be understood that one or several leaflets of the leaflet assembly will remain movably attached to other parts of the leaflet assembly even after attachment so as to provide a physiological function after implantation.

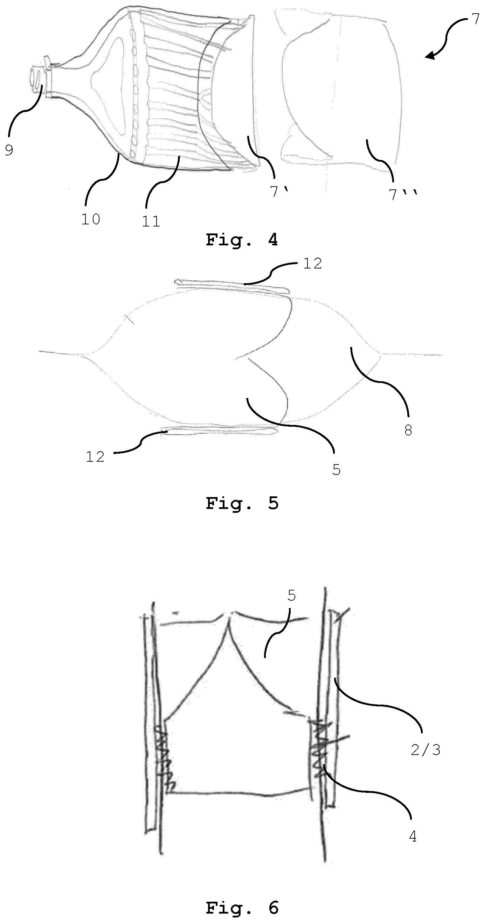

Fig. 4 shows a further embodiment of a mold 7, similar to the embodiment shown in Figs. 2a-2c. Hier, the first part 7' has channels 11 adapted for delivery of an adhesive composition. A Luer interface 9 is provided at one end of the first part 7'. Through the Luer interface 9, a commercially available syringe may be connected for delivering the adhesive composition to the channels. Surrounding the mold 7 is a shrink tube 10 (e.g. a heat shrinkable tube). When the leaflet assembly (not shown here) is formed, a stent (not shown) may be placed around the leaflet assembly with the shrink tube 10 on the outside of the stent. Through channels 11, the adhesive can be delivered while the shrink tube compresses the assembly.

Fig. 5 shows a further method of providing an adhesive on a leaflet assembly 5. Here, a pocket 12 is arranged circumferentially around the leaflet assembly 5. The pocket 12 contains an adhesive composition and is adapted to disintegrate when friction is applied to it, e.g. by comprising or consisting of a brittle material. A balloon 8 is used to exert a force from inside to outside, thereby also creating friction against a stent (not shown) which may be arranged circumferentially outside leaflet assembly 5 and pockets 12. Additionally, the stent and leaflet assembly 5 may rotated with respect to one another to create more friction. When the pockets 12 disintegrate, the adhesive composition is released. Additionally or alternatively, adhesive capsule may be arranged in the pockets 12 and/or directly on the outside surface of leaflet assembly 5. The balloon 8 may be formed by a commercially available True^{™} Flow catheter.

Fig. 6 shows a main body 2 formed by a stent structure coated with pericardium tissue. A cyanoacrylate adhesive 4 is used to attach the leaflet assembly 5 to the pericardium layer 3 on the main body 2.

## Claims

1. A medical implant (1), preferably an implantable valve, comprising a main body (2) and a leaflet assembly (5), wherein the leaflet assembly (5) is formed separate from the main body (2) and is attachable or attached thereto.

2. The medical implant (1) according to claim 1, wherein the main body (2) is a stent.

3. The medical implant (1) according to any one of the preceding claims, wherein the main body (2) comprises or consists of a material selected from a shape memory alloy, a resorbable metal alloy, a bimetal, a magnesium alloy, an iron alloy, a cobalt alloy, a chromium alloy, a polymer, in particular a polylactide, and/or a combination thereof.

4. The medical implant (1) according to any one of the preceding claims, wherein the main body (2) is adapted to be placed in one of an aortic valve, a mitral valve, a pulmonary valve, and/or a tricuspid valve.

5. The medical implant (1) according to any one of the preceding claims, wherein the leaflet assembly (5) is an autologous leaflet assembly (5), preferably obtainable by a process comprising the steps of providing human or animal body tissue; shaping the body tissue in a shaping process to give the body tissue a shape and size of a cardiac valve; fixation and stabilization of the body tissue by a cross-linking agent, thereby preserving the shape given to the body tissue by the shaping process.

6. The medical implant (1) according to any one of the preceding claims, wherein the main body (2) is coated with a material (3) selected from electrospun fibers, polymers, synthetic tissues, pericardium, peritoneum, fascia lata, and/or a material applied by an electrospinning and/or dip coating and/or spray coating process, and/or a combination thereof.

7. The medical implant (1) according to any one of the preceding claims, wherein the leaflet assembly (5) is attachable or attached to the main body (2) and/or a main body coating by an adhesive connection (4).

8. The medical implant (1) according to claim 7, wherein the adhesive connection (4) is formed by an adhesive selected from a methacrylated gelatine, a cyanoacrylate, a fibrin-based adhesive, an adhesive powder, collagen-based adhesives, or a combination thereof.

9. A method of producing a medical implant (1), preferably a medical implant (1) according to any one of the preceding claims, comprising the steps of
a. Providing a main body (2);
b. Providing a leaflet assembly (5);
c. Attaching the leaflet assembly (5) to the main body (2), preferably by glueing with an adhesive composition (4).

10. The method according to claim 9, further comprising a step of producing a leaflet assembly (5) by providing human or animal body tissue; shaping the body tissue in a shaping process to give the body tissue a shape and size of a cardiac valve; fixation and stabilization of the body tissue by a cross-linking agent, thereby preserving the shape given to the body tissue by the shaping process.

11. The method according to any one of claims 9 to 10, further comprising a step of curing the adhesive composition (4).

12. A method of treating a patient, comprising the steps of
a. Obtaining body tissue from the patient to be treated,
b. shaping the body tissue in a shaping process to give the body tissue a shape and size of a leaflet assembly (5) ;
c. fixating and stabilizing of the body tissue by a cross-linking agent, thereby preserving the shape given to the body tissue by the shaping process,
d. attaching the leaflet assembly (5) to an implant main body (2), preferably by glueing, to form a medical implant (1), preferably a medical implant (1) according to any one of the claims 1 to 8;
e. implanting the medical implant (1) in the patient.

13. The method according to claim 12, wherein the medical implant (1) is a prosthetic cardiac valve, and the medical implant (1) is implanted in the patient at a native cardiac valve.

14. The method according to any one of claims 12 or 13, further comprising the step of removing the implant main body (2) after implantation.
